# EUROPEAN PATENT SPECIFICATION

(11) **EP 4 274 488 B1**
(45) Date of publication and mention of the grant of the patent: **13.11.2024**
(21) Application number: 22701481.8
(22) Date of filing: 06.01.2022
(51) Int. Cl.: A61B 10/02

(54) **BIOPSY APPARATUS HAVING MULTIPLE AIR PATHS FOR VENTING**
BIOPSIEVORRICHTUNG MIT MEHREREN LUFTWEGEN ZUR ENTLÜFTUNG
APPAREIL DE BIOPSIE AYANT DE MULTIPLES TRAJETS D'AIR POUR VENTILATION

(30) Priority: 08.01.2021 US 202163135256 P
(43) Date of publication of application: 15.11.2023
(73) Proprietor: Bard Peripheral Vascular, Inc., Franklin Lakes, NJ 07417 (US)
(72) Inventor: ORTS, Soren F., 2830 Virum (DK); FRACZKOWSKI, Marek, 70-783 Szczecin (PL)
(74) Representative: Hoffmann Eitle
(86) International application number: PCT/US2022/011453
(87) International publication number: WO 2022/150487

(56) References cited:
- WO-A1-2020/093635
- US-A1- 2005 080 355
- US-A1- 2019 117 201
- US-A1- 2020 187 921

## Description

### BACKGROUND

### 1. Field of the Disclosure

The present disclosure relates to biopsy devices, and, more particularly, to a single insertion multiple sample biopsy apparatus.

### 2. Description of the Related Art

A biopsy may be performed on a patient to help in determining whether the tissue in a region of interest includes cancerous cells. One biopsy technique used to evaluate breast tissue, for example, involves inserting a biopsy probe into the breast tissue region of interest to capture one or more tissue samples from the region. Such a biopsy technique often utilizes a vacuum to pull the tissue to be sampled into a sample notch of the biopsy probe, after which the tissue is severed and collected. Efforts continue in the art to improve the ability of the biopsy device to sever a tissue sample, and to transport the severed tissue sample to a sample collection container.

What is needed in the art is a biopsy device that has the ability to promote effective severing of a tissue sample and effective transport of the tissue sample to a sample collection container.
US 2019/117201 A1 discloses a biopsy device including coaxially disposed inner and outer needles in which the outer needle tip is configured for obtaining a tissue sample. The inner surface of the outer needle includes a tissue retention feature which includes a countersink having a predetermined length from the distal cutting edge of the outer needle.
US 2005/080355 A1 discloses a needle set for a biopsy device. The needle set includes an outer member, a cylinder lumen within said outer member, an inner member having a cannula and an inner lumen. The inner member is slidably disposed within the cylinder lumen. The biopsy device also includes a cylinder seal member that is disposed within the cylinder lumen and a cannula seal member attached to the outer surface of the cannula. The cylinder seal member and the cannula seal member define a vacuum chamber therebetween.
WO 2020/093635 A1 discloses a biopsy rotary cutting device, comprising a dynamic sealing structure provided outside an inner knife tube. The dynamic sealing structure forms a cavity outside the inner knife tube. The inner knife tube comprises a first tube section and a second tube section that are sequentially arranged in the axial direction of the inner knife tube. The outer diameter of the second tube section is less than the outer diameter of the first tube section. The dynamic sealing structure is internally provided with a seal ring. The cavity comprises a first cavity and a second cavity. When the inner knife tube moves to a first position along the axial direction thereof, the seal ring is outside the second tube section, and there is an inter-cavity gap between the seal ring and the second tube section, to communicate the first cavity with the second cavity.
US 2020/187921 A1 on which the preamble of claim 1 is based *+ discloses a biopsy apparatus including a biopsy probe assembly that is releasably attached to a driver assembly. The biopsy probe assembly has a vacuum cannula and a stylet cannula coaxially arranged. The vacuum cannula has a flared portion that extends distally from an elongate portion. The stylet cannula is movable between a first extended position and a first retracted position. The stylet cannula has a distal portion having a sample notch and a protrusion member that extends proximally in a lumen of stylet cannula along a portion of a longitudinal extent of the sample notch, wherein when the stylet cannula is in the first retracted position, the protrusion member is received within the flared portion of the vacuum cannula. The biopsy apparatus may further include a controller circuit that has a virtual energy reservoir, and the controller circuit executes program instructions to control current to motors when engaging dense tissue.

### SUMMARY

In one embodiment a biopsy apparatus, including a biopsy probe assembly having a cutter cannula, a vacuum cannula, and a stylet cannula coaxially arranged along a longitudinal axis. The vacuum cannula is positioned inside the stylet cannula to define a first intermediate lumen therebetween. The stylet cannula is positioned within the cutter cannula to define a second intermediate lumen therebetween. The vacuum cannula has a vacuum lumen. The stylet cannula is movable relative to the vacuum cannula and the cutter cannula between a first extended position and a first retracted position. The stylet cannula has a plurality of vent openings, the plurality of vent openings including at least one longitudinal vent slot. The biopsy apparatus also includes a seal having a proximal seal portion and a distal seal portion, the distal seal portion having a distal seal lip that is positioned for radial engagement with an outside diameter of the cutter cannula, and the proximal seal portion having a proximal seal lip that is positioned for radial engagement with an outside diameter of the stylet cannula. When the stylet cannula is moved from the first extended position toward the first retracted position, the at least one longitudinal vent slot is longitudinally positioned under the proximal seal lip of the proximal seal portion of the seal so as to open a seal bypass path across a longitudinal extent of the proximal seal lip of the proximal seal portion of the seal so as to establish both a first air path at the first intermediate lumen between an outside diameter of the vacuum cannula and an inside diameter of the stylet cannula and a second air path at the second intermediate lumen between an inside diameter of the cutter cannula and the outside diameter of the stylet cannula. The first air path and the second air path are in fluid communication with a region proximal to the seal.

In another embodiment not forming part of the invention as claimed, a method includes applying vacuum via a vacuum cannula of a biopsy apparatus to a lumen of a stylet cannula of the biopsy apparatus at a sample notch of the stylet cannula. A cutter cannula of the biopsy apparatus, the vacuum cannula, and the stylet cannula, are coaxially arranged along a longitudinal axis. The vacuum cannula is positioned inside the stylet cannula to define a first intermediate lumen therebetween, the stylet cannula is positioned within the cutter cannula to define a second intermediate lumen therebetween, and the vacuum cannula has a vacuum lumen. The stylet cannula is movable relative to the vacuum cannula and the cutter cannula between a first extended position and a first retracted position. The stylet cannula has a plurality of vent openings, the plurality of vent openings including at least one longitudinal vent slot. The biopsy apparatus includes a seal having a proximal seal portion and a distal seal portion, the distal seal portion having a distal seal lip that is positioned for radial engagement with an outside diameter of the cutter cannula, and the proximal seal portion having a proximal seal lip that is positioned for radial engagement with an outside diameter of the stylet cannula. The method also includes moving the stylet cannula toward the first retracted position to move a severed tissue sample into the vacuum cannula. When the stylet cannula is moved toward the first retracted position, the at least one longitudinal vent slot is longitudinally positioned under the proximal seal lip of the proximal seal portion of the seal so as to open a seal bypass path across a longitudinal extent of the proximal seal lip of the proximal seal portion of the seal so as to establish both a first air path at the first intermediate lumen between an outside diameter of the vacuum cannula and an inside diameter of the stylet cannula and a second air path at the second intermediate lumen between an inside diameter of the cutter cannula and the outside diameter of the stylet cannula. The first air path and the second air path are in fluid communication with a region proximal to the seal.

These and additional features provided by the embodiments described herein will be more fully understood in view of the following detailed description, in conjunction with the drawings.

### BRIEF DESCRIPTION OF THE DRAWINGS

The above-mentioned and other features and advantages of this disclosure, and the manner of attaining them, will become more apparent and the disclosure will be better understood by reference to the following description of an embodiment of the disclosure taken in conjunction with the accompanying drawings, wherein:
Fig. 1 is a perspective view of a biopsy apparatus configured in accordance with an embodiment of the present disclosure, with a biopsy probe assembly attached to a biopsy driver assembly;
Fig. 2 is a perspective view of the biopsy apparatus of Fig. 1, with the biopsy probe assembly detached from the biopsy driver assembly and with an upper cover of the biopsy driver assembly removed to expose the piercing module of the biopsy driver assembly;
Fig. 2A is a bottom view of the biopsy driver assembly of Fig. 2, with the biopsy driver assembly inverted from the orientation shown in Fig. 2;
Fig. 3 is a block representation of the biopsy driver assembly of Fig. 1;
Fig. 4 is an exploded view of the biopsy probe assembly of Fig. 1;
Fig. 5A is a section view of the biopsy probe assembly of Fig. 1, taken along line 5A-5A of Fig. 2;
Fig. 5B is an enlarged portion of the vacuum cannula depicted in Fig. 5A;
Fig. 5C is an enlarged portion of the stylet cannula depicted in Fig. 5A;
Fig. 6A shows the relative positions of the vacuum cannula, the stylet cannula, and the cutter cannula before, during, and immediately after a piercing shot;
Fig. 6B shows the relative positions of the vacuum cannula, the stylet cannula, and the cutter cannula, and with the cutter cannula retracted to expose the sample notch of the stylet cannula;
Fig. 6C shows the relative positions of the vacuum cannula, the stylet cannula, and the cutter cannula, depicting a shaking of the sample notch by alternatingly moving the stylet cannula in the proximal direction and in the distal direction for a short distance;
Fig. 6D shows the relative positions of the vacuum cannula, the stylet cannula, and the cutter cannula, wherein the cutter cannula is rotated and translated in the distal direction to sever a tissue sample from the tissue received in the sample notch;
Fig. 6E shows the relative positions of the vacuum cannula, the stylet cannula, and the cutter cannula, wherein the stylet cannula is moved within the cutter cannula in the proximal direction to mechanically aid in moving the tissue sample into the flared portion of the vacuum cannula;
Fig. 6F shows the relative positions of the vacuum cannula, the stylet cannula, and the cutter cannula, wherein the stylet cannula is moved within the cutter cannula in the distal direction to disengage the protrusion member from the flared portion of the vacuum cannula;
Fig. 6G shows the relative positions of the vacuum cannula, the stylet cannula, and the cutter cannula, wherein the stylet cannula is again moved within the cutter cannula in the proximal direction, such that the protrusion member re-engages the flared portion of the vacuum cannula;
Fig. 6H shows the relative positions of the vacuum cannula, the stylet cannula, and the cutter cannula, wherein the stylet cannula is again moved within the cutter cannula in the distal direction to disengage the protrusion member from the flared portion of the vacuum cannula and return to the extended position;
Fig. 7 is a vacuum/time graph (normalized) depicting a baseline vacuum pressure at several different positions during a tissue sample cutting and transport sequence as depicted in Figs. 6A-6H;
Fig. 8 is a perspective view of the stylet cannula showing a longitudinal vent slot of two diametrically opposed longitudinal vent slots;
Fig. 8A is a section view of the stylet cannula taken along line 8A-8A of Fig. 8 depicting an arrangement of vent openings including two diametrically opposed longitudinal vent slots and a plurality of circular vent holes;
Fig. 9 is a side view of the biopsy probe assembly of Fig. 2 that includes an introducer cannula, and with the stylet cannula substantially positioned as in Fig. 6D;
Fig. 10 is an enlarged section view of a portion of the biopsy probe assembly taken along line 10-10 of Fig. 9;
Fig. 11 is a further enlargement of a portion of the section view of Fig. 10 depicting the first and second air paths;
Fig. 12 is a side view of the biopsy probe assembly of Fig. 2 that includes an introducer cannula, and with the stylet cannula substantially positioned as in Fig. 6E;
Fig. 13 is an enlarged section view of a portion of the biopsy probe assembly taken along line 13-13 of Fig. 12;
Fig. 14 is a further enlargement of a portion of the section view of Fig. 13 depicting the first and second air paths; and
Fig. 15 is a section view of a portion of the biopsy probe assembly of Fig. 2, with the protrusion member of the piercing tip of the stylet cannula positioned slightly distal to the retracted position depicted in Fig. 6E, and depicting a combined air flow formed from the combining of the air flows from the first and second air paths.

Corresponding reference characters indicate corresponding parts throughout the several views. The exemplifications set out herein illustrate at least one embodiment of the disclosure, and such exemplifications are not to be construed as limiting the scope of the application in any manner.

### DESCRIPTION OF EMBODIMENTS

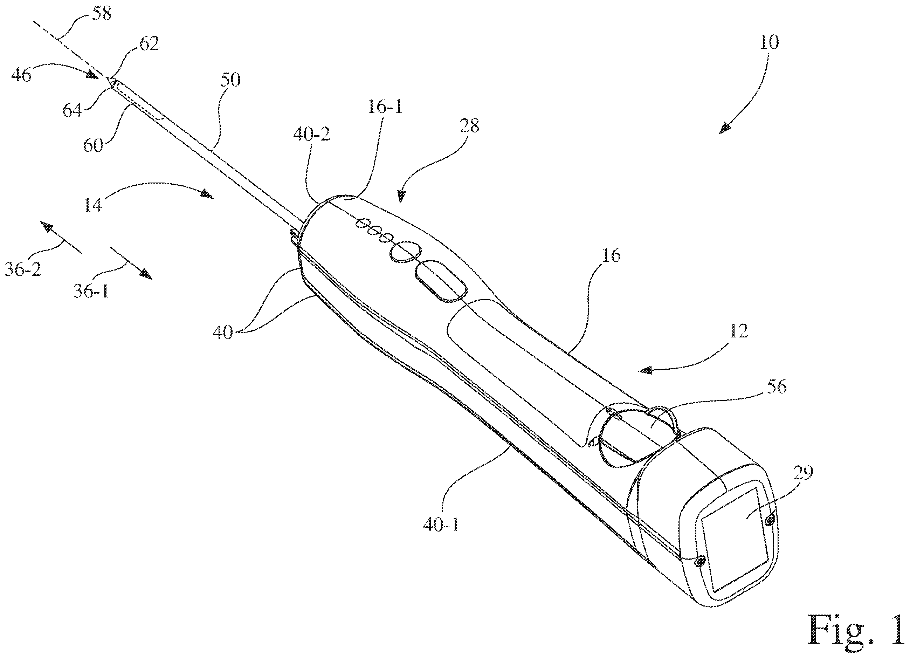

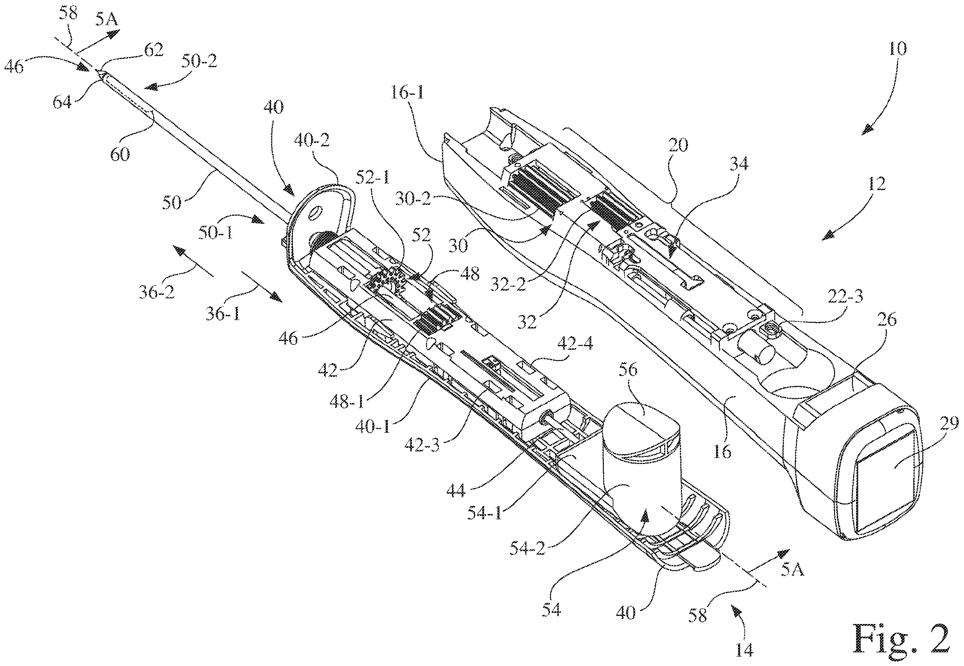

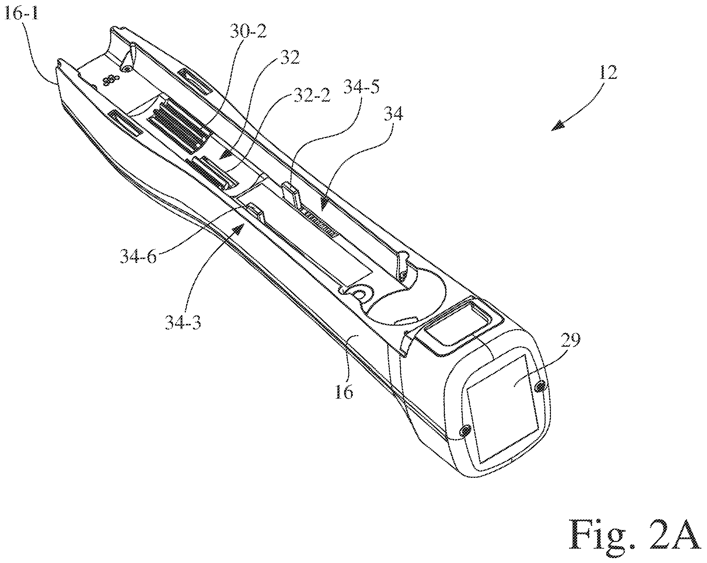

Referring now to the drawings, and more particularly to Figs. 1 and 2, there is shown a biopsy apparatus 10 which generally includes a non-invasive, e.g., non-disposable, biopsy driver assembly 12 and an invasive, e.g., disposable, biopsy probe assembly 14. As used herein, the term "non-disposable" is used to refer to a device that is intended for use on multiple patients during the lifetime of the device, and the term "disposable" is used to refer to a device that is intended to be disposed of after use on a single patient. Biopsy driver assembly 12 includes a driver housing 16 that is configured and ergonomically designed to be grasped by a user.

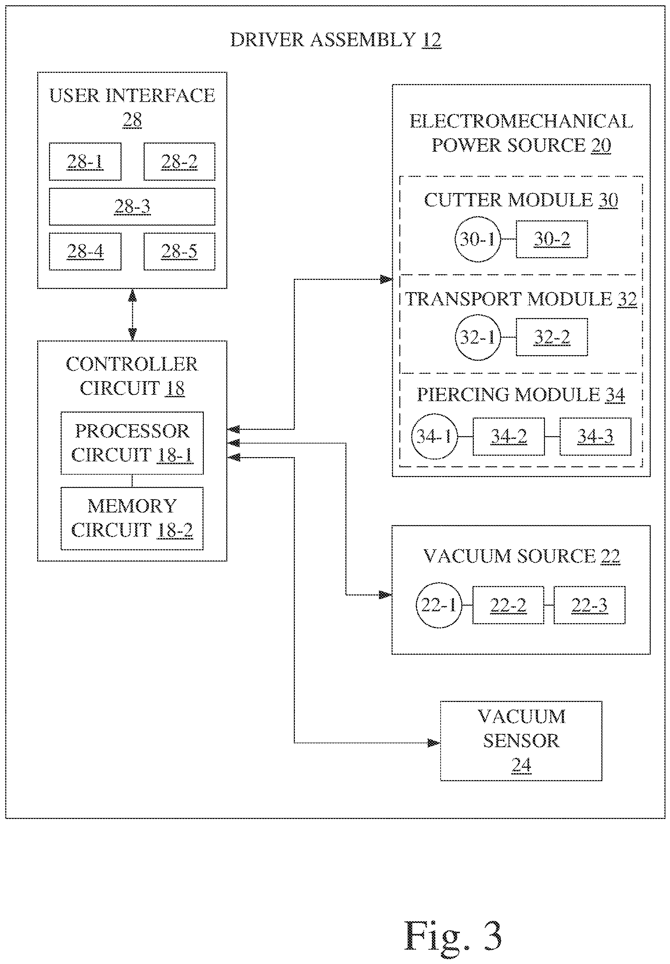

Referring to Figs. 2 and 3, biopsy driver assembly 12 includes within driver housing 16 a controller circuit 18, an electromechanical power source 20, a vacuum source 22, a vacuum sensor 24, and a battery 26 (or alternatively an AC adapter). A user interface 28 (see Fig. 1), such as a keypad, is located to be mounted to driver housing 16, and externally accessible by the user with respect to driver housing 16. Battery 26 may be, for example, a rechargeable battery, which may be charged by an inductive charging device coupled to inductive coil 29, or alternatively, by an electrical connection to an electrical power supply. Battery 26 is electrically coupled to controller circuit 18, electromechanical power source 20, vacuum source 22, and user interface 28.

Referring to Fig. 3, user interface 28 may include control buttons and visual/aural indicators, with the control buttons providing user control over various functions of biopsy apparatus 10, and with the visual/aural indicators providing visual/aural feedback of the status of one or more conditions and/or positions of components of biopsy apparatus 10. The control buttons may include a sample button 28-1 and a prime/pierce button 28-2. The visual indicators may include a display screen 28-3 and/or one or more light emitting diodes (LED) 28-4. The aural indicator may include a buzzer 28-5. The control buttons may include tactile feedback to the user when activated.

Controller circuit 18 is electrically and communicatively coupled to electromechanical power source 20, vacuum source 22, vacuum sensor 24, and user interface 28, such as by one or more wires or circuit traces. Controller circuit 18 may be assembled on an electrical circuit board, and includes, for example, a processor circuit 18-1 and a memory circuit 18-2.

Processor circuit 18-1 has one or more programmable microprocessors and associated circuitry, such as an input/output interface, clock, buffers, memory, etc. Memory circuit 18-2 is communicatively coupled to processor circuit 18-1, e.g., via a bus circuit, and is a non-transitory electronic memory that may include volatile memory circuits, such as random access memory (RAM), and non-volatile memory circuits, such as read only memory (ROM), electronically erasable programmable ROM (EEPROM), NOR flash memory, NAND flash memory, etc. Controller circuit 18 may be formed as one or more Application Specific Integrated Circuits (ASIC).

Controller circuit 18 is configured via software and/or firmware residing in memory circuit 18-2 to execute program instructions to perform functions associated with the retrieval of biopsy tissue samples, such as that of controlling and/or monitoring one or more components of electromechanical power source 20, vacuum source 22, and vacuum sensor 24.

Electromechanical power source 20 may include, for example, a cutter module 30, a transport module 32, and a piercing module 34, each being respectively electrically coupled to battery 26. Each of cutter module 30, transport module 32, and piercing module 34 is electrically and controllably coupled to controller circuit 18 by one or more electrical conductors, e.g., wires or circuit traces.

Cutter module 30 may include an electrical motor 30-1 having a shaft to which a drive gear 30-2 is attached. Transport module 32 may include an electrical motor 32-1 having a shaft to which a drive gear 32-2 is attached. Piercing module 34 may include an electrical motor 34-1, a drive spindle 34-2, and a piercing shot drive 34-3. Each electrical motor 30-1, 32-1, 34-1 may be, for example, a direct current (DC) motor or stepper motor. As an alternative to the arrangement described above, each of cutter module 30, transport module 32, and piercing module 34 may include one or more of a gear, gear train, belt/pulley arrangement, etc., interposed between the respective motor and drive gear or drive spindle.

Piercing module 34 is configured such that an activation of electrical motor 34-1 and a drive spindle 34-2 causes a piercing shot drive 34-3 to move in a proximal direction 36-1 to compress a firing spring, e.g., one or more coil springs, and to latch piercing shot drive 34-3 in a ready position. Upon actuation of prime/pierce button 28-2 of user interface 28, piercing shot drive 34-3 is propelled, i.e., fired, in a distal direction 36-2 (see Fig. 2).

Vacuum source 22 is electrically and controllably coupled to battery 26 by one or more electrical conductors, e.g., wires or circuit traces. Vacuum source 22 may include, for example, an electric motor 22-1 that drives a vacuum pump 22-2. Vacuum source 22 has a vacuum source port 22-3 coupled to vacuum pump 22-2 for establishing vacuum in biopsy probe assembly 14. Electric motor 22-1 may be, for example, a rotary, linear or vibratory DC motor. Vacuum pump 22-2 may be, for example, a peristaltic pump or a diaphragm pump, or one or more of each connected in series or parallel.

Vacuum sensor 24 is electrically coupled to controller circuit 18 by one or more electrical conductors, e.g., wires or circuit traces. Vacuum sensor 24 may be a pressure differential sensor that provides vacuum (negative pressure) feedback signals to controller circuit 18. In some implementations, vacuum sensor 24 may be incorporated into vacuum source 22.

Referring to Figs. 1 and 2, biopsy probe assembly 14 is configured for releasable attachment to biopsy driver assembly 12. As used herein, the term "releasable attachment" means a configuration that facilitates an intended temporary connection followed by selective detachment involving a manipulation of disposable biopsy probe assembly 14 relative to biopsy driver assembly 12, without the need for tools.

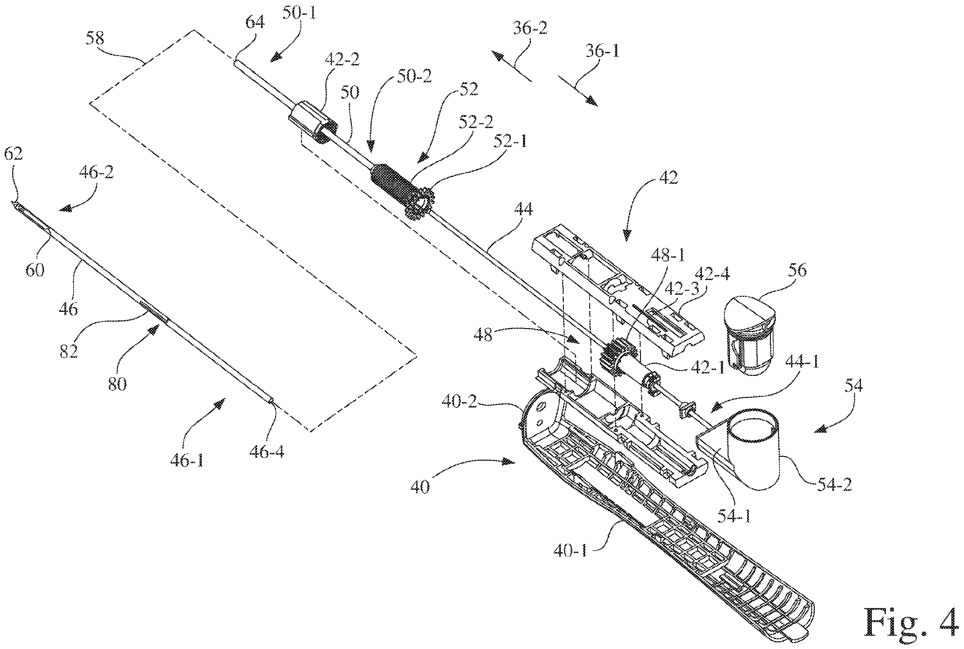

Referring to the exploded view of Fig. 4, biopsy probe assembly 14 includes a probe housing 40, a probe sub-housing 42, a vacuum cannula 44, a stylet cannula 46, a stylet gear-spindle set 48 for linear stylet translation, a cutter cannula 50, a cutter gear-spindle set 52 for rotary and linear cutter translation, a sample manifold 54, and a sample cup 56.

Referring to Figs. 2, 4, and 5A, probe housing 40 is formed as an L-shaped structure having an elongate portion 40-1 and a front plate 40-2. When biopsy probe assembly 14 is attached to biopsy driver assembly 12, front plate 40-2 is positioned distally adjacent to an entirety of front surface 16-1 of driver housing 16, i.e., so as to shield the entirety of front surface 16-1 of the non-disposable driver assembly from contact with a patient.

Vacuum cannula 44, stylet cannula 46, and cutter cannula 50 are coaxially arranged along a longitudinal axis 58 in a nested tube arrangement, with vacuum cannula 44 being the innermost tube, cutter cannula 50 being the outermost tube, and stylet cannula 46 being the intermediate tube that is interposed between vacuum cannula 44 and cutter cannula 50. In other words, vacuum cannula 44 is positioned inside stylet cannula 46, and stylet cannula 46 is positioned inside cutter cannula 50.

Vacuum cannula 44 is mounted to be stationary relative to probe sub-housing 42. Vacuum cannula 44 is coupled in fluid communication with vacuum source 22 via sample manifold 54.

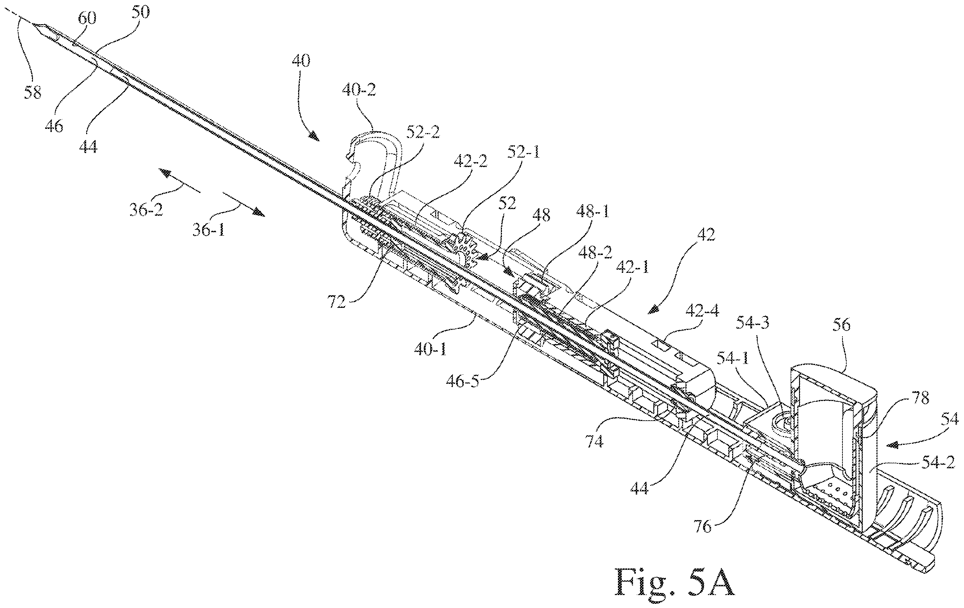

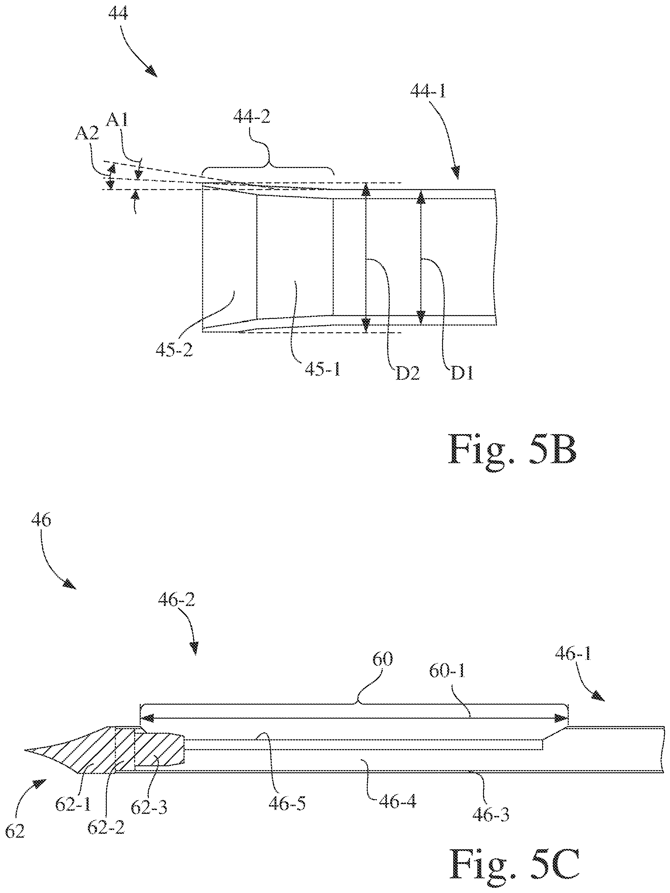

Referring to Figs. 4, 5A, and 5B, vacuum cannula 44 includes an elongate portion 44-1, a flared portion 44-2 that extends distally from elongate portion 44-1, and a vacuum lumen 44-3. Elongate portion 44-1 has a first outside diameter D1. Flared portion 44-2 flares from elongate portion 44-1 in two stages, namely, a first flared stage 45-1 and a second flared stage 45-2. First flared stage 45-1 diverges from elongate portion 44-1 at a first acute angle A1, and second flared stage 45-2 diverges from first flared stage 45-1 at a second acute angle A2 relative to elongate portion 44-1, with acute angle A2 being larger than acute angle A1. A distal outside diameter D2 of second flared stage 45-2 is selected to be accommodated within, and in sliding contact with, lumen 46-4 of stylet cannula 46. Each of first flared stage 45-1 and second flared stage 45-2 of flared portion 44-2 has a distally and gradually increasing diameter, which is larger than the diameter D1 of elongate portion 44-1.

Referring to Figs. 4 and 5A, stylet cannula 46 includes a proximal portion 46-1 and a distal portion 46-2. Distal portion 46-2 includes a sample notch 60. Attached to distal portion 46-2 is a piercing tip 62, which in turn forms part of stylet cannula 46. Stylet gear-spindle set 48 threadably engages external threads of a transport spindle 46-5 that is fixedly attached (e.g., glued, welded or staked) to proximal portion 46-1 of stylet cannula 46. Stylet gear-spindle set 48 is a unitary gear having a driven gear 48-1 fixedly attached to a threaded spindle 48-2, and may be formed as a single molded component. Stylet cannula 46 is retracted or extended along longitudinal axis 58 by activation of transport module 32 of biopsy probe assembly 14, with drive gear 32-2 of transport module 32 of biopsy driver assembly 12 being engaged with driven gear 48-1 of stylet gear-spindle set 48.

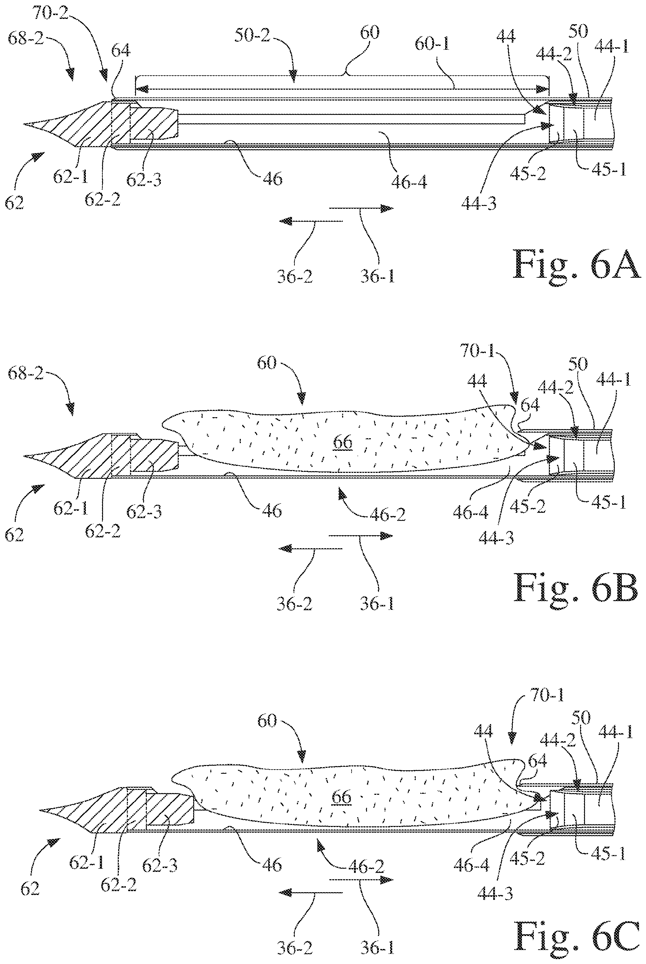

Referring also to Fig. 5C, 6A, and 6B, sample notch 60 is formed as an elongate opening in a side wall 46-3 of stylet cannula 46 to facilitate a reception of tissue 66 into a lumen 46-4 of stylet cannula 46. Sample notch 60 has a longitudinal extent 60-1 that extends along longitudinal axis longitudinal axis 58. Sample notch 60 does not extend in side wall 46-3 below a centerline of the diameter of stylet cannula 46, and may include cutting edges around the perimeter of the opening formed by sample notch 60, wherein the cutting edges of the elongate (linear) portions of sample notch 60 each have a cutting edge that diverges from a cutting edge along the side wall 46-3 to the centerline at a diameter of stylet cannula 46.

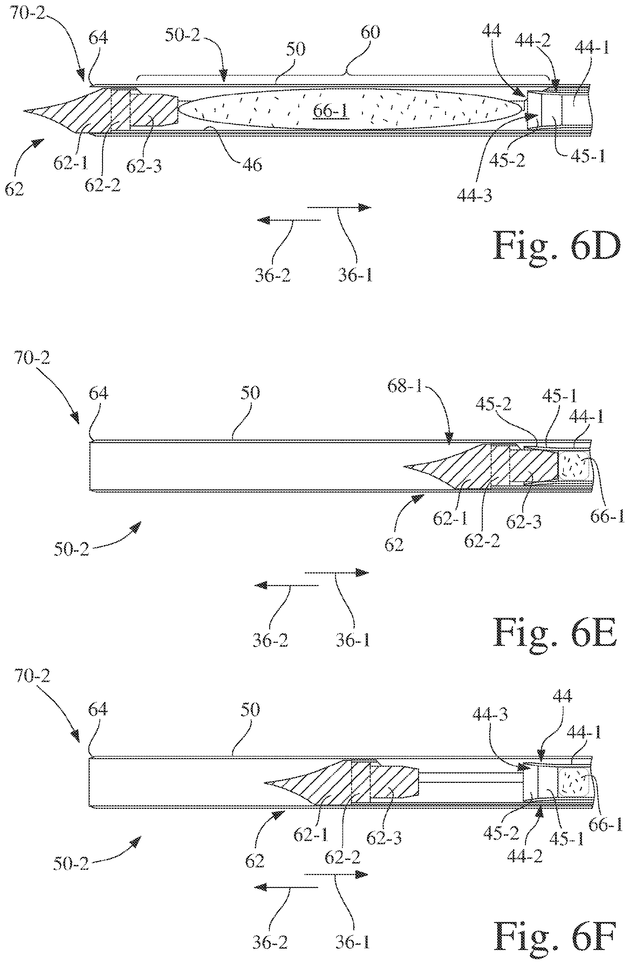

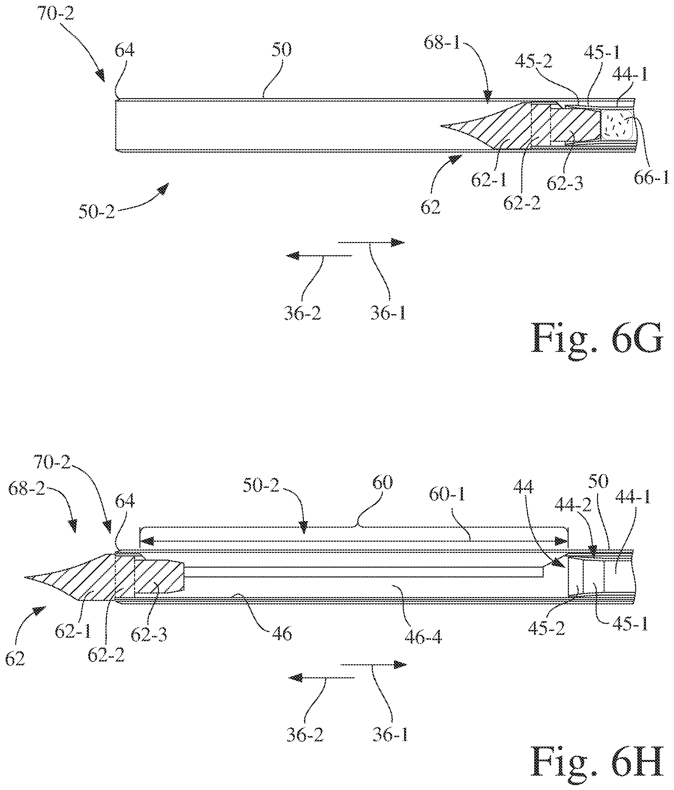

Piercing tip 62 has a tip portion 62-1, a mounting portion 62-2, and a protrusion member 62-3. Piercing tip 62 is inserted into lumen 46-4 of stylet cannula 46 at distal portion 46-2, with mounting portion 62-2 being attached to distal portion 46-2 of stylet cannula 46, such as an adhesive or weld. As such, tip portion 62-1 extends distally from distal portion 46-2 of stylet cannula 46, and protrusion member 62-3 extends proximally (i.e., in proximal direction 36-1) in lumen 46-4 along a portion of the longitudinal extent 60-1 of sample notch 60. Accordingly, as depicted in Figs. 6E and 6G, when stylet cannula 46 is fully retraced in the proximal direction 36-1, protrusion member 62-3 is received into flared portion 44-2 of vacuum cannula 44. At least the proximal tip portion of protrusion member 62-3 has a proximally decreasing diameter.

Referring again to Fig. 4, cutter cannula 50 includes a proximal portion 50-1 and a distal portion 50-2. Distal portion 50-2 includes an annular cutting edge 64. Cutter gear-spindle set 52 is fixedly attached (e.g., glued, welded or staked) to proximal portion 50-1 of cutter cannula 50. Cutter gear-spindle set 52 is a unitary gear having a driven gear 52-1 fixedly attached to a threaded spindle 52-2, and may be formed as a single molded component. Cutter cannula 50 is retracted or extended along longitudinal axis 58 by activation of cutter module 30 of biopsy probe assembly 14, with drive gear 30-2 of cutter module 30 of biopsy driver assembly 12 being engaged with driven gear 52-1 of cutter gear-spindle set 52. Thus, cutter cannula 50 has a rotational cutting motion and is translated axially along longitudinal axis 58. The pitch of the threads of threaded spindle 52-2 determines the number of revolutions per axial distance (in millimeters (mm)) that cutter cannula 50 moves axially.

Referring to Figs. 4 and 5A, sample manifold 54 is configured as an L-shaped structure having a vacuum chamber portion 54-1 and a collection chamber portion 54-2. Vacuum chamber portion 54-1 includes a vacuum input port 54-3 that is arranged to sealably engage vacuum source port 22-3 of vacuum source 22 of biopsy driver assembly 12 when biopsy probe assembly 14 is attached to biopsy driver assembly 12. Vacuum chamber portion 54-1 is connected in fluid communication with collection chamber portion 54-2. Proximal end of elongate portion 44-1 of vacuum cannula 44 passes through vacuum chamber portion 54-1 and is in direct fluid communication with collection chamber portion 54-2. Collection chamber portion 54-2 has a cavity sized and arranged to removably receive sample cup 56, such that sample cup 56 is in direct fluid communication with elongate portion 44-1 of vacuum cannula 44, and sample cup 56 also is in direct fluid communication with vacuum input port 54-3 of vacuum chamber portion 54-1. Blotting papers are placed in vacuum chamber portion 54-1 in a region between vacuum input port 54-3 and collection chamber portion 54-2.

Accordingly, a tissue sample severed by cutter cannula 50 at sample notch 60 of stylet cannula 46 may be transported by vacuum applied by vacuum source 22 at sample cup 56, through vacuum cannula 44, and into sample cup 56.

Referring again to Fig. 2, 4 and 5A, probe sub-housing 42, e.g., a probe carrier body, is a sub-housing that is slidably coupled to probe housing 40, e.g., using a rail/slot arrangement. Probe sub-housing 42 includes a proximal threaded portion 42-1 and a distal threaded portion 42-2. Also, probe sub-housing 42 is configured to be drivably coupled to piercing module 34. Stated differently, piercing module 34 is drivably coupled to probe sub-housing 42 when biopsy driver assembly 12 is coupled to biopsy probe assembly 14 to form biopsy apparatus 10.

Probe sub-housing 42 includes one or more piercing module engagement opening, e.g., slot(s). In the present embodiment, with reference to Figs. 2 and 2A, probe sub-housing 42 includes a piercing module engagement opening 42-3 and a piercing module engagement opening 42-4, each of which is configured, e.g., in size and in shape, to receive a respective drive protrusion 34-5, 34-6 of piercing shot drive 34-3 of piercing module 34 so as to effect longitudinal movement of probe sub-housing 42 in unison with longitudinal movement of piercing shot drive 34-3 during a piercing shot (firing) operation. For example, each of piercing module engagement opening 42-3 and piercing module engagement opening 42-4 may be a respective rectangular slot. While the present embodiment includes two piercing module engagement openings for symmetry and/or redundancy, an alternative embodiment may have, for example, only one piercing module engagement opening, e.g., piercing module engagement opening 42-3.

Proximal threaded portion 42-1 in probe sub-housing 42 has a threaded hole that threadably receives threaded spindle 48-2 of stylet gear-spindle set 48, such that rotation of driven gear 48-1 of stylet gear-spindle set 48 results in a linear translation of stylet cannula 46 along longitudinal axis 58, with a direction of rotation correlating to a direction of translation of stylet cannula 46 in one of proximal direction 36-1 and distal direction 36-2. Driven gear 48-1 of stylet gear-spindle set 48 engages drive gear 32-2 of transport module 32 when biopsy probe assembly 14 is attached to biopsy driver assembly 12 (see Fig. 1).

Likewise, distal threaded portion 42-2 of probe sub-housing 42 has a threaded hole that threadably receives threaded spindle 52-2 of cutter gear-spindle set 52, such that rotation of driven gear 52-1 of cutter gear-spindle set 52 results in a combined rotation and linear translation of cutter cannula 50 along longitudinal axis 58, with a direction of rotation correlating to a direction of translation of cutter cannula 50. Driven gear 52-1 of cutter gear-spindle set 52 engages drive gear 30-2 of cutter module 30 when biopsy probe assembly 14 is attached to biopsy driver assembly 12 (see Fig. 1).

Also, when biopsy probe assembly 14 is attached to biopsy driver assembly 12, referring also to Figs. 2 and 3, probe sub-housing 42 is connected to piercing shot drive 34-3 of piercing module 34. As such, upon a first actuation of prime/pierce button 28-2, probe sub-housing 42 and piercing shot drive 34-3 are translated in unison in proximal direction 36-1 to position piercing shot drive 34-3 and probe sub-housing 42 carrying stylet cannula 46 and cutter cannula 50 in the ready, i.e., cocked position, and upon a second actuation of prime/pierce button 28-2 to effect a piercing shot, probe sub-housing 42 and piercing shot drive 34-3 are rapidly propelled in unison in distal direction 36-2 to position stylet cannula 46 and cutter cannula 50 at the distal most position of the combined elements, e.g., within the patient.

Figs. 6A-6H collectively represent a tissue sample severing and transport sequence. Figs. 6E and 6G show stylet cannula 46 in its retracted position 68-1. Figs. 6A, 6B, and 6H show stylet cannula 46 in its extended position 68-2, sometimes also referred to as a zero position. Figs. 6C, 6D, and 6F show stylet cannula 46 in various positions intermediate to retracted position 68-1 and extended position 68-2. Figs. 6B and 6C show cutter cannula 50 in its retracted position 70-1, which exposes sample notch 60 of stylet cannula 46 when stylet cannula 46 is in or near its extended position 68-2. Figs. 6A and 6D-6H show cutter cannula 50 in its extended position 70-2, sometimes also referred to as a zero position, wherein cutter cannula 50 covers the sample notch 60 of stylet cannula 46.

To effect the described movements of stylet cannula 46, controller circuit 18 executes program instructions and sends respective control signals to transport module 32 of biopsy driver assembly 12, which in turn transfers the motion to stylet gear-spindle set 48 of biopsy probe assembly 14. Likewise, to effect the described movements of cutter cannula 50, controller circuit 18 executes program instructions and sends respective control signals to cutter module 30 of biopsy driver assembly 12, which in turn transfers the motion to cutter gear-spindle set 52 of biopsy probe assembly 14. Controller circuit 18 may determine an axial position of each of stylet cannula 46 and cutter cannula 50, relative to the respective zero position, by counting the respective number of motor drive pulses, or alternatively, the respective number of motor shaft revolutions.

Fig. 6A shows the relative positions of vacuum cannula 44, stylet cannula 46, and cutter cannula 50 before, during, and immediately after the piercing shot effected by piercing module 34. As shown, distal portion 50-2 of cutter cannula 50 is extended over sample notch 60.

In the sequence step illustrated in Fig. 6B, vacuum source 22 is actuated to deliver a vacuum via vacuum cannula 44 to lumen 46-4 of stylet cannula 46 at sample notch 60, and cutter cannula 50 is retracted by actuation of cutter module 30 to expose sample notch 60, thereby permitting tissue 66 to be drawn into lumen 46-4 of stylet cannula 46 through sample notch 60. In the present embodiment, in order to expose sample notch 60, cutter cannula 50 rotates counterclockwise to effect a linear translation of cutter cannula 50 in proximal direction 36-1 for a distance of approximately 23 millimeters (mm) to define the open length of sample notch 60. As used herein, the relative term "approximately" means the base value in the indicated units (if any) plus or minus five percent, unless stated otherwise. The actual aperture size at sample notch 60, corresponding to a desired sample size, may be user-selected at user interface 28, wherein a distance that cutter cannula 50 is retracted toward retracted position 70-1 from extended position 70-2 is controlled by controller circuit 18 to correspond to the sample size selected by the user.

Figs. 6C and 6D illustrate a cutting sequence.

In the sequence step illustrated in Fig. 6C, in order to increase the size of tissue sample to be collected, stylet cannula 46 may be moved alternatingly in proximal direction 36-1 and distal direction 36-2 a short distance e.g., 2 to 5 mm, so as to shake sample notch 60, thereby increasing the amount of tissue 66 that passes through sample notch 60 and into lumen 46-4 of stylet cannula 46. The last move of the shake is defined to keep sample notch 60 in a 1 mm retracted position (see Fig. 6C) compared to the zero position of stylet cannula 46 as depicted in Fig. 6A. This is to ensure that cutter cannula 50 closes sample notch 60 during the cutting sequence (see Fig. 6D) and will cut 1 mm further, to thus ensure that connective tissue or strings are completely cut during the cutting sequence step illustrated in Fig 6D.

In the cutting sequence step illustrated in Fig. 6D, cutter cannula 50 is rotated and translated in distal direction 36-2 to sever a tissue sample 66-1 from tissue 66. In the present embodiment, cutter cannula 50 rotates clockwise to effect a linear translation of cutter cannula in distal direction 36-2 for a distance of approximately 23 mm in order to cut the tissue and return to the zero position.

Figs. 6E-6H illustrate a tissue sample transport sequence.

In the sequence step illustrated in Fig. 6E, vacuum is applied by vacuum cannula 44, and stylet cannula 46 is moved within cutter cannula 50 in proximal direction 36-1 to mechanically aid in moving tissue sample 66-1 into flared portion 44-2 of vacuum cannula 44. More particularly, as stylet cannula 46 is moved within cutter cannula 50 in proximal direction 36-1, protrusion member 62-3 of piercing tip 62 engages tissue sample 66-1 to assist tissue sample 66-1 into vacuum cannula 44. Protrusion member 62-3 then engages flared portion 44-2 of vacuum cannula 44 to close off an air inflow into flared portion 44-2 of vacuum cannula 44.

In the sequence step illustrated in Fig. 6F, with vacuum being applied by vacuum cannula 44, stylet cannula 46 is moved within cutter cannula 50 in distal direction 36-2 to disengage protrusion member 62-3 from the flared portion 44-2 of vacuum cannula 44 to cause an abrupt change in air flow into vacuum cannula 44, thereby helping the vacuum transport of tissue sample 66-1 through vacuum cannula 44.

The sequence steps illustrated in Figs. 6G and 6H are essentially a repeat of sequence steps 6E and 6F.

In the sequence step illustrated in Fig. 6G, with vacuum applied to vacuum cannula 44 by vacuum source 22, stylet cannula 46 is again moved within cutter cannula 50 in proximal direction 36-1, such that protrusion member 62-3 of piercing tip 62 re-engages flared portion 44-2 of vacuum cannula 44 to again close off an air inflow into flared portion 44-2 of vacuum cannula 44.

In the sequence step illustrated in Fig. 6H, with vacuum being applied to vacuum cannula 44 by vacuum source 22, stylet cannula 46 is moved within cutter cannula 50 in distal direction 36-2 to again disengage protrusion member 62-3 from flared portion 44-2 of vacuum cannula 44 to cause an abrupt change in air flow into vacuum cannula 44, thereby helping the vacuum transport of tissue sample 66-1 (if not already delivered by sequence steps of Figs. 6E and 6F) through vacuum cannula 44. At the end of the sequence of Fig. 6H, stylet cannula 46 is re-positioned at the tissue receiving position i.e., extended position 68-2, also referred to as the zero position, and is ready to receive tissue for a next tissue sample, in which the sequence steps of Figs. 6A-6H would be repeated.

It is noted that the sample transport sequence illustrated in Figs. 6E and 6F may be repeated as many times as necessary to complete the vacuum transport of tissue sample 66-1 through vacuum cannula 44. Also, the backward motion of protrusion member 62-3 of piercing tip 62 of stylet cannula 46 in proximal direction 36-1 may be implemented as incremental steps, alternating between a backward motion and then a forward motion (the forward distance being less than the backward distance) until the final position (retracted position 68-1) is reached, as depicted in Figs. 6E and 6G.

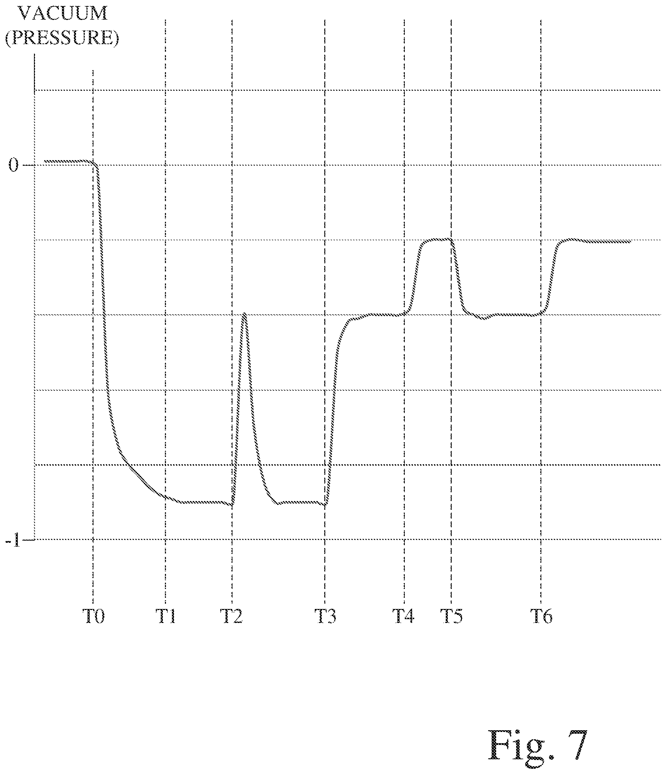

Fig. 7 is a vacuum graph (normalized) depicting a baseline vacuum pressure at different positions during the tissue sample cutting and transport sequence depicted in Figs. 6A-6H.

Referring to the vacuum graph of Fig. 7, it is noted that vacuum is applied throughout the entire sequence depicted in Figs. 6A-6H. At time T0, vacuum source 22 is activated, and vacuum (negative pressure) builds in vacuum cannula 44. At time T1, maximum vacuum is achieved, which corresponds to the end of the cutting sequence step depicted in Fig. 6D. At time T2, the tissue stuffing sequence of Figs. 6E-6F begins, and vacuum pressure abruptly drops due to a moment in which vent openings 80 in stylet cannula 46 are not restricted. Vacuum begins to build prior to time T3 as protrusion member 62-3 of piercing tip 62 approaches flared portion 44-2 of vacuum cannula 44, and maximizes, representing the end of the first stuffing sequence depicted in Fig. 6E. At time T3, vacuum pressure abruptly drops due to protrusion member 62-3 of piercing tip 62 being moved away from flared portion 44-2 as depicted in Fig. 6F. In some instances, tissue sample 66-1 may have been delivered to sample cup 56. At time T4, the second stuffing sequence depicted in Figs. 6G and 6H begins. Time T5 corresponds to the end of the second stuffing sequence depicted in Fig. 6G. At time T6, vacuum pressure drops due to protrusion member 62-3 of piercing tip 62 again being moved away from flared portion 44-2 as depicted in Fig. 6H, and back to the tissue receiving (zero) position.

By comparing an actual vacuum pressure to the baseline vacuum graph depicted in Fig. 7 at different stages of the tissue cutting and transport sequence depicted in Figs. 6A-6H, cutting or tissue transport anomalies can be identified and corrective action can be attempted.

In accordance with an aspect of the disclosure, vacuum sensor 24 provides vacuum pressure feedback signals to controller circuit 18, and controller circuit 18 executes program instructions to determine whether the actual vacuum pressure provided by vacuum sensor 24 deviates by more than a predetermined amount from the baseline pressure of the vacuum graph of Fig. 7 at a corresponding point in the tissue cutting and transport sequence. The predetermined amount may be, for example, the baseline vacuum pressure plus or minus 10 percent. If the deviation is outside the acceptable range of deviation, then corrective action may be taken depending upon when in the tissue cutting and transport sequence the anomaly occurred.

For example, if the vacuum pressure falls below the baseline by more than the allowable deviation during the time period between times T1 and T2, this may be an indication of an incomplete cut, and thus controller circuit 18 may repeat the cutting sequence depicted in Figs. 6C and 6D without user intervention, rather than immediately going into an error state. Similarly, if the vacuum pressure rises above the baseline by more than the allowable deviation between the times T3 to T5, this may be an indication of an incomplete tissue transport through vacuum cannula 44, and thus controller circuit 18 may increase the number of iterations of sequence steps 6E and 6F without user intervention.

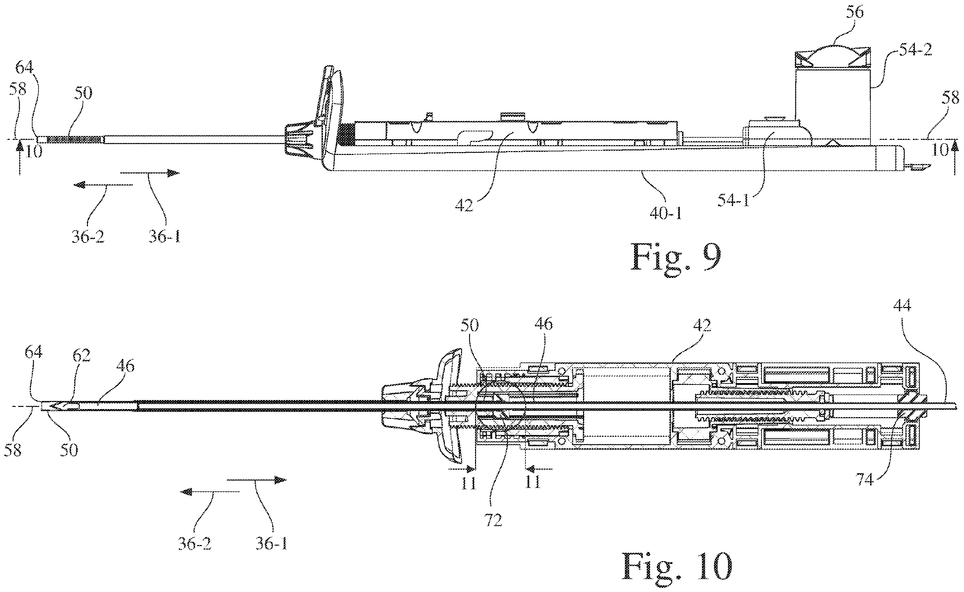

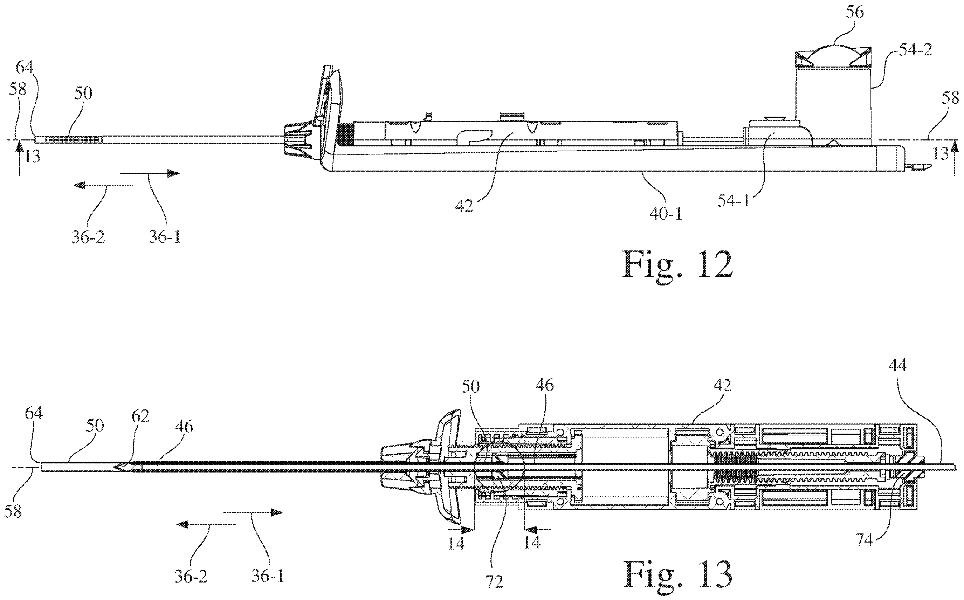

Referring again to Fig. 5A in conjunction with Figs. 10 and 13, vacuum is maintained in biopsy probe assembly 14 by a series of seals. A seal 72, e.g., a sleeve-type seal, is located to provide a seal between cutter cannula 50 and stylet cannula 46. A seal 74, e.g., an O-ring seal, is located to provide a seal between stylet cannula 46 and vacuum cannula 44. A seal 76, e.g., a sleeve-type seal or O-ring arrangement, is located to provide a seal between vacuum cannula 44 and vacuum chamber portion 54-1 of sample manifold 54. Also, a seal 78 may be located in collection chamber portion 54-2 of sample manifold 54 and sample cup 56. Finally, a seal is placed at vacuum input port 54-3 at the vacuum interface between biopsy probe assembly 14 and biopsy driver assembly 12.

During operation, vacuum pump 22-2 of vacuum source 22 will build up vacuum (negative pressure) in the vacuum reservoir formed by sample manifold 54 and sample cup 56. More particularly, the volume of sample cup 56 and sample manifold 54 will define the strength of a "vacuum boost", and also defines the cycle time for vacuum pump 22-2 of vacuum source 22. In the present embodiment, for example, the volume is approximately 25 milliliters.

Regarding the "vacuum boost", stylet cannula 46 has one or more vent openings 80 longitudinally separated from sample notch 60. Vent openings 80 may be annularly arranged at a predetermined distance proximal from sample notch 60 and tip portion 62-1, and these vent openings 80 (see Fig. 4) will be exposed to the atmosphere when the stylet cannula 46 is retracted to retracted position 68-1 (see Figs. 6E and 6G), wherein vent openings 80 slide under seal 72 between cutter cannula 50 and stylet cannula 46. Once these vent openings 80 are exposed to the atmosphere, the system is 'open' and the build-up vacuum pressure will be equalized with the surrounding pressure so as to create the vacuum boost effect, in addition to the continuous flow delivered by vacuum pump 22-2 of vacuum source 22.

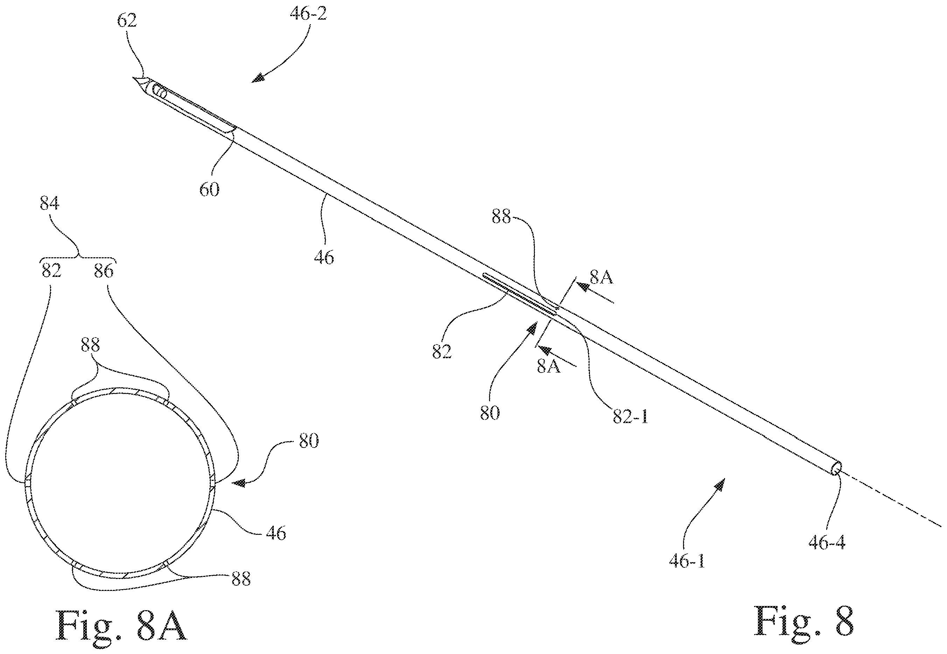

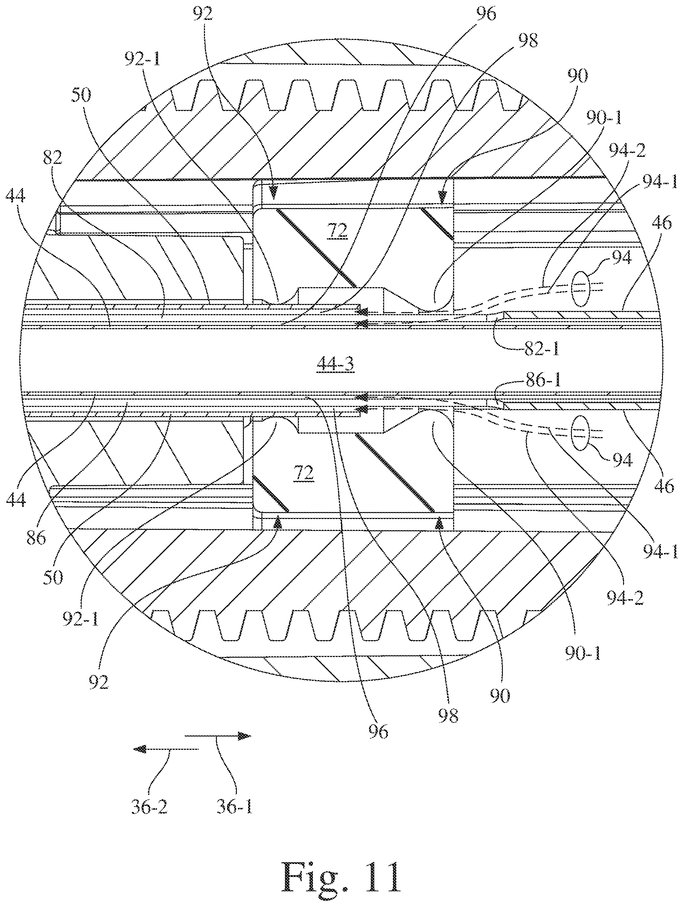

Referring also to Figs. 8 and 8A, in the present embodiment, vent openings 80 in stylet cannula 46 include at least one longitudinal vent slot 82, and in the present embodiment, may include a plurality of longitudinal vent slots 84 (e.g., longitudinal vent slot 82 and longitudinal vent slot 86) and a plurality of circular vent holes 88, e.g., two or more circular vent holes. In the present embodiment, longitudinal vent slot 82 and longitudinal vent slot 86 are diametrically opposed. Also, in the present embodiment, the proximal ends of the plurality of longitudinal vent slots 84, e.g., proximal end 82-1 of longitudinal vent slot 82 and proximal end 86-1 of longitudinal vent slot 86 (see also Fig. 11), are longitudinally aligned with the plurality of circular vent holes 88.

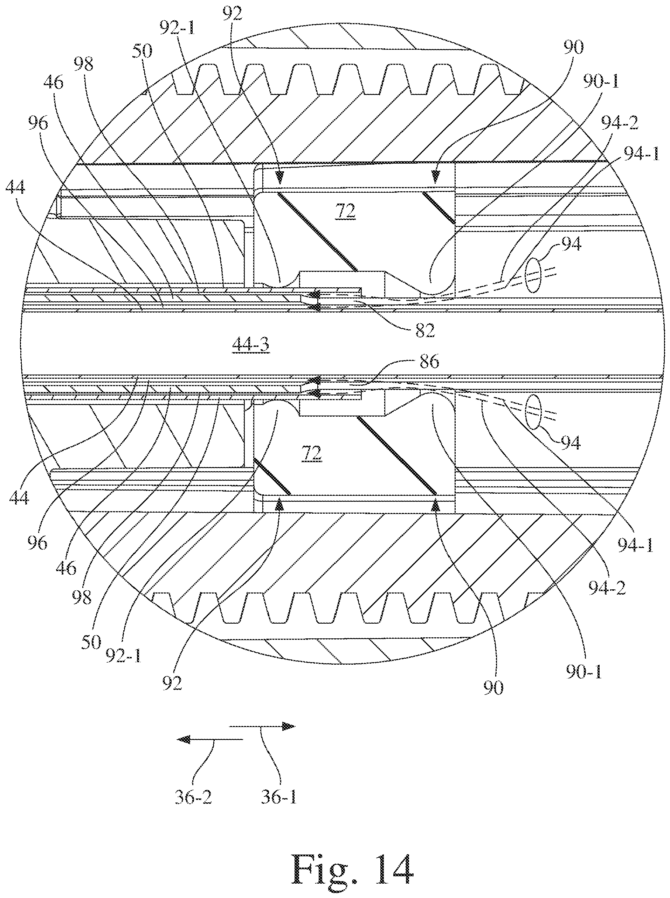

With reference particularly to Figs. 11 and 14, seal 72 includes a proximal seal portion 90 and a distal seal portion 92. Distal seal portion 92 of seal 72 includes a distal seal lip 92-1 that is positioned for radial engagement with the outside diameter (OD) of cutter cannula 50. Proximal seal portion 90 of seal 72 includes a proximal seal lip 90-1 that is positioned for radial engagement with the outside diameter (OD) of stylet cannula 46.

Each longitudinal vent slot, e.g., the plurality of longitudinal vent slots 84, of stylet cannula 46 is configured, in size and in shape, to facilitate an increased air flow to vacuum lumen 44-3 of vacuum cannula 44 over that of a corresponding number of circular vent holes, so as to aid in movement of the severed tissue sample 66-1 (see also Figs. 6E-6G) in proximal direction 36-1. In the present embodiment, for example, each longitudinal vent slot 82, 86 of the plurality of longitudinal vent slots 84 of stylet cannula 46 may have a longitudinal length of 21 millimeters and a width of 0.75 millimeters.

More particularly, with reference to Figs. 9-14, as stylet cannula 46 is retracted (see Figs. 6D-6E) in proximal direction 36-1 toward retracted position 68-1, each of the plurality of longitudinal vent slots 84 of stylet cannula 46 open a seal bypass path 94 (see Figs. 11 and 14) across the longitudinal extent of proximal seal lip 90-1 of proximal seal portion 90 of seal 72 so as to establish both a first air path 94-1 at an intermediate lumen 96 between the outside diameter (OD) of vacuum cannula 44 and the inside diameter (ID) of stylet cannula 46 and a second air path 94-2 at an intermediate lumen 98 between the ID of cutter cannula 50 and the OD of stylet cannula 46. Each of first air path 94-1 and second air path 94-2 is in fluid communication with the atmosphere in a region proximal to seal 72 when seal bypass path 94 is established, i.e., by positioning at least one longitudinal vent slot, e.g., longitudinal vent slot 82 and/or longitudinal vent slot 86, of stylet cannula 46 to longitudinally bridge across the proximal seal lip 90-1 of proximal seal portion 90 of seal 72.

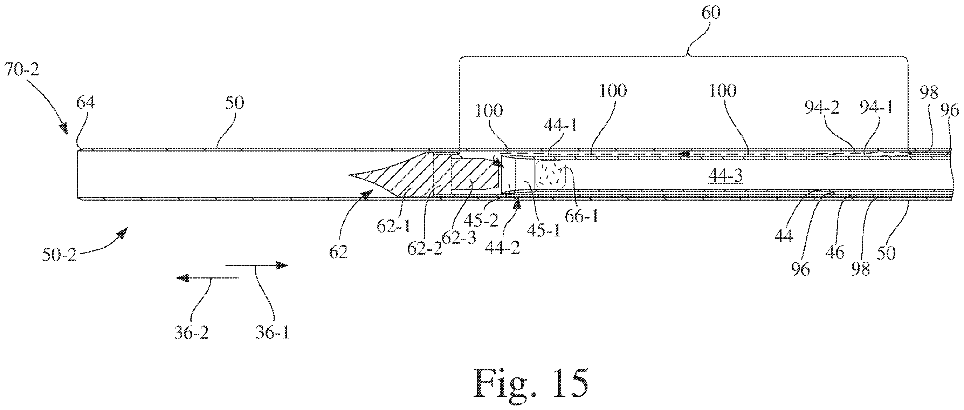

With further reference to Fig. 15, first air path 94-1 and second air path 94-2 converge at sample notch 60 of stylet cannula 46 to establish a combined air flow 100 of first air path 94-1 and second air path 94-2 to vacuum lumen 44-3 of vacuum cannula 44, thus facilitating an increased air flow to vacuum lumen 44-3 of vacuum cannula 44 over that of the use of the plurality of circular vent holes 88 (e.g., six) without the use of longitudinal vent slots. Stated differently, Fig. 15 shows that with the use of at least one longitudinal vent slot 82, and in the present embodiment, a plurality of longitudinal vent slots 84, more air will flow through and from both of intermediate lumen 96 and intermediate lumen 98, wherein the combined air flow 100 reaches the tissue sample 66-1, and then pressure equalization will happen much faster and stronger as more air can be moved into vacuum lumen 44-3 of vacuum cannula 44 due to the additional (second) air path 94-2 so as to move tissue sample 66-1 through vacuum lumen 44-3 of vacuum cannula 44. In the absence of the at least one longitudinal slot 82 and/or longitudinal vent slot 86 of stylet cannula 46, the additional (second) air path 94-2 would not be established by the arrangement of components of the present embodiment.

As used herein, "substantially", "slightly", "approximately" and other words of degree are relative modifiers intended to indicate permissible variation from the characteristic so modified. It is not intended to be limited to the absolute value or characteristic which it modifies but rather possessing more of the physical or functional characteristic than its opposite, and approaching or approximating such a physical or functional characteristic.

Also, as used herein, the term "coupled", and its derivatives, is intended to embrace any operationally functional connection, i.e., a direct connection or an indirect connection.

While this application has been described with respect to at least one embodiment, the present application can be further modified within scope of the invention as claimed.

This application is therefore intended to cover any variations, uses, or adaptations within the scope of the claims.

## Claims

1. A biopsy apparatus (10), comprising:
a biopsy probe assembly (14) having a cutter cannula (50), a vacuum cannula (44), and a stylet cannula (46) coaxially arranged along a longitudinal axis (58), the vacuum cannula (44) being positioned inside the stylet cannula (46) to define a first intermediate lumen (96) therebetween, the stylet cannula (46) being positioned within the cutter cannula (50) to define a second intermediate lumen (98) therebetween, and the vacuum cannula (44) having a vacuum lumen (44-3), wherein:
the stylet cannula (46) is movable relative to the vacuum cannula (44) and the cutter cannula (50) between a first extended position (68-2) and a first retracted position (68-1); and
the stylet cannula (46) has a plurality of vent openings (80), the plurality of vent openings (80) including at least one longitudinal vent slot (82, 86); and
a seal (72) having a proximal seal portion (90) and a distal seal portion (92), the distal seal portion (92) having a distal seal lip (92-1) that is positioned for radial engagement with an outside diameter of the cutter cannula (50), and the proximal seal portion (90) having a proximal seal lip (90-1) that is positioned for radial engagement with an outside diameter of the stylet cannula (46), wherein:
when the stylet cannula (46) is moved from the first extended position (68-2) toward the first retracted position (68-1), the at least one longitudinal vent slot (82, 86) is longitudinally positioned under the proximal seal lip (90-1) of the proximal seal portion (90) of the seal (72) so as to open a seal bypass path (94) across a longitudinal extent of the proximal seal lip (90-1) of the proximal seal portion (90) of the seal (72) so as to establish both a first air path (94-1) at the first intermediate lumen (96) between an outside diameter of the vacuum cannula (44) and an inside diameter of the stylet cannula (46) and a second air path (94-2) at the second intermediate lumen (98) between an inside diameter of the cutter cannula (50) and the outside diameter of the stylet cannula (46), the first air path (94-1) and the second air path (94-2) being in fluid communication with a region proximal to the seal (72).

2. The biopsy apparatus (10) of claim 1, further comprising a driver assembly (12) having an electromechanical power source (20) and a vacuum source (22), wherein the biopsy probe assembly (14) is releasably attached to the driver assembly (12).

3. The biopsy apparatus (10) of claim 1, further comprising a driver assembly (12) having an electromechanical power source (20) and a vacuum source (22), wherein the vacuum lumen (44-3) of the vacuum cannula (44) is coupled in fluid communication with the vacuum source (22).

4. The biopsy apparatus (10) of claim 1, further comprising a driver assembly (12) having an electromechanical power source (20) and a vacuum source (22), wherein the stylet cannula (46) is coupled in driving communication with the electromechanical power source (20).

5. The biopsy apparatus (10) of claim 1, further comprising a driver assembly (12) having an electromechanical power source (20) and a vacuum source (22), wherein the cutter cannula (50) is coupled in driving communication with the electromechanical power source (20).

6. The biopsy apparatus (10) of claim 1, wherein the cutter cannula (50) is movable relative to the stylet cannula (46) between a second extended position (70-2) to cover a sample notch (60) of the stylet cannula (46) and a second retracted position (70-1) to expose the sample notch (60) when the stylet cannula (46) is in the first extended position (68-2).

7. The biopsy apparatus (10) of claim 1, wherein the stylet cannula (46) has a proximal portion (46-1) and a distal portion (46-2), the distal portion (46-2) defining a sample notch (60).

8. The biopsy apparatus (10) of claim 7, wherein the plurality of vent openings (80) are arranged at a location proximal to the sample notch (60).

9. The biopsy apparatus (10) of claim 7, wherein the first air path (94-1) and the second air path (94-2) converge at the sample notch (60).

10. The biopsy apparatus (10) of claim 1, wherein the plurality of vent openings (80) includes at least two longitudinal vent slots (84).

11. The biopsy apparatus (10) of claim 10, wherein a first longitudinal vent slot (82) and a second longitudinal vent slot (86) are diametrically opposed.

12. The biopsy apparatus (10) of claim 1, wherein the plurality of vent openings (80) includes at least one circular vent hole (88).

13. The biopsy apparatus (10) of claim 12, wherein a proximal end (82-1, 86-1) of the at least one longitudinal vent slot (82, 86) is aligned with the at least one circular vent hole (88).

## Patentansprüche

1. Biopsievorrichtung (10), umfassend:
eine Biopsiesondenanordnung (14), die eine Schneidkanüle (50), eine Vakuumkanüle (44) und eine Stilettkanüle (46) aufweist, die koaxial entlang einer Längsachse (58) angeordnet sind, wobei die Vakuumkanüle (44) im Inneren der Stilettkanüle (46) positioniert ist, um dazwischen ein erstes Zwischenlumen (96) zu definieren, wobei die Stilettkanüle (46) innerhalb der Schneidkanüle (50) positioniert ist, um dazwischen ein zweites Zwischenlumen (98) zu definieren, und wobei die Vakuumkanüle (44) ein Vakuumlumen (44-3) aufweist, wobei:
die Stilettkanüle (46) relativ zu der Vakuumkanüle (44) und der Schneidkanüle (50) zwischen einer ersten ausgefahrenen Position (68-2) und einer ersten eingefahrenen Position (68-1) beweglich ist; und
die Stilettkanüle (46) eine Vielzahl von Entlüftungsöffnungen (80) aufweist, wobei die Vielzahl von Entlüftungsöffnungen (80) mindestens einen longitudinalen Entlüftungsschlitz (82, 86) einschließt; und
eine Dichtung (72), die einem proximalen Dichtungsabschnitt (90) und einen distalen Dichtungsabschnitt (92) aufweist, wobei der distale Dichtungsabschnitt (92) eine distale Dichtungslippe (92-1) aufweist, die für einen radialen Eingriff mit einem Außendurchmesser der Schneidkanüle (50) positioniert ist, und wobei der proximale Dichtungsabschnitt (90) eine proximale Dichtungslippe (90-1) aufweist, die für einen radialen Eingriff mit einem Außendurchmesser der Stilettkanüle (46) positioniert ist, wobei:
wenn die Stilettkanüle (46) von der ersten ausgefahrenen Position (68-2) in Richtung der ersten eingefahrenen Position (68-1) bewegt wird, der mindestens eine longitudinale Entlüftungsschlitz (82, 86) longitudinal unter der proximalen Dichtungslippe (90-1) des proximalen Dichtungsabschnitts (90) der Dichtung (72) positioniert ist, um einen Dichtungsbypassweg (94) über eine longitudinale Erstreckung der proximalen Dichtungslippe (90-1) des proximalen Dichtungsabschnitts (90) der Dichtung (72) zu öffnen, um sowohl einen ersten Luftweg (94-1) am ersten Zwischenlumen (96) zwischen einem Außendurchmesser der Vakuumkanüle (44) und einem Innendurchmesser der Stilettkanüle (46) als auch einen zweiten Luftweg (94-2) am zweiten Zwischenlumen (98) zwischen einem Innendurchmesser der Schneidkanüle (50) und dem Außendurchmesser der Stilettkanüle (46) herzustellen, wobei der erste Luftweg (94-1) und der zweite Luftweg (94-2) mit einem Bereich proximal zur Dichtung (72) in Fluidverbindung stehen.

2. Biopsievorrichtung (10) nach Anspruch 1, weiter umfassend eine Antriebsanordnung (12), die eine elektromechanische Leistungsquelle (20) und eine Vakuumquelle (22) aufweist, wobei die Biopsiesondenanordnung (14) lösbar an der Antriebsanordnung (12) angebracht ist.

3. Biopsievorrichtung (10) nach Anspruch 1, weiter umfassend eine Antriebsanordnung (12), die eine elektromechanische Leistungsquelle (20) und eine Vakuumquelle (22) aufweist, wobei das Vakuumlumen (44-3) der Vakuumkanüle (44) mit der Vakuumquelle (22) in Fluidverbindung gekoppelt ist.

4. Biopsievorrichtung (10) nach Anspruch 1, weiter umfassend eine Antriebsanordnung (12), die eine elektromechanische Leistungsquelle (20) und eine Vakuumquelle (22) aufweist, wobei die Stilettkanüle (46) mit der elektromechanischen Leistungsquelle (20) in Antriebsverbindung gekoppelt ist.

5. Biopsievorrichtung (10) nach Anspruch 1, weiter umfassend eine Antriebsanordnung (12), die eine elektromechanische Leistungsquelle (20) und eine Vakuumquelle (22) aufweist, wobei die Schneidkanüle (50) mit der elektromechanischen Leistungsquelle (20) in Antriebsverbindung gekoppelt ist.

6. Biopsievorrichtung (10) nach Anspruch 1, wobei, wenn sich die Stilettkanüle (46) in der ersten ausgefahrenen Position (68-2) befindet, die Schneidkanüle (50) zwischen einer zweiten ausgefahrenen Position (70-2) zum Abdecken einer Probenkerbe (60) der Stilettkanüle (46) und einer zweiten eingefahrenen Position (70-1) zum Freilegen der Probenkerbe (60) relativ zur Stilettkanüle (46) beweglich ist.

7. Biopsievorrichtung (10) nach Anspruch 1, wobei die Stilettkanüle (46) einen proximalen Abschnitt (46-1) und einen distalen Abschnitt (46-2) aufweist, wobei der distale Abschnitt (46-2) eine Probenkerbe (60) definiert.

8. Biopsievorrichtung (10) nach Anspruch 7, wobei die Vielzahl von Entlüftungsöffnungen (80) an einer Stelle proximal zur Probenkerbe (60) angeordnet ist.

9. Biopsievorrichtung (10) nach Anspruch 7, wobei der erste Luftweg (94-1) und der zweite Luftweg (94-2) an der Probenkerbe (60) zusammenlaufen.

10. Biopsievorrichtung (10) nach Anspruch 1, wobei die Vielzahl von Entlüftungsöffnungen (80) mindestens zwei longitudinale Entlüftungsschlitze (84) einschließt.

11. Biopsievorrichtung (10) nach Anspruch 10, wobei ein erster longitudinaler Entlüftungsschlitz (82) und ein zweiter longitudinaler Entlüftungsschlitz (86) diametral gegenüberliegen.

12. Biopsievorrichtung (10) nach Anspruch 1, wobei die Vielzahl von Entlüftungsöffnungen (80) mindestens ein kreisförmiges Entlüftungsloch (88) einschließt.

13. Biopsievorrichtung (10) nach Anspruch 12, wobei ein proximales Ende (82-1, 86-1) des mindestens einen longitudinalen Entlüftungsschlitzes (82, 86) mit dem mindestens einen kreisförmigen Entlüftungsloch (88) ausgerichtet ist.

## Revendications

1. Appareil de biopsie (10), comprenant :
un ensemble sonde de biopsie (14) ayant une canule de coupe (50), une canule sous vide (44), et une canule à stylet (46) disposées coaxialement le long d'un axe longitudinal (58), la canule sous vide (44) étant positionnée à l'intérieur de la canule à stylet (46) pour définir une première lumière intermédiaire (96) entre elles, la canule à stylet (46) étant positionnée à l'intérieur de la canule de coupe (50) pour définir une seconde lumière intermédiaire (98) entre elles, et la canule sous vide (44) ayant une lumière sous vide (44-3), dans lequel :
la canule à stylet (46) est mobile par rapport à la canule sous vide (44) et à la canule de coupe (50) entre une première position étendue (68-2) et une première position rétractée (68-1) ; et la canule à stylet (46) présente une pluralité d'ouvertures d'évent (80), la pluralité d'ouvertures d'évent (80) incluant au moins une fente d'évent longitudinale (82, 86) ; et
un joint (72) ayant une partie de joint proximale (90) et une partie de joint distale (92), la partie de joint distale (92) ayant une lèvre de joint distale (92-1) qui est positionnée pour une mise en prise radiale avec un diamètre extérieur de la canule de coupe (50), et la partie de joint proximale (90) ayant une lèvre de joint proximale (90-1) qui est positionnée pour une mise en prise radiale avec un diamètre extérieur de la canule à stylet (46), dans lequel :
lorsque la canule à stylet (46) est déplacée de la première position étendue (68-2) vers la première position rétractée (68-1), l'au moins une fente d'évent longitudinale (82, 86) est positionnée longitudinalement sous la lèvre de joint proximale (90-1) de la partie de joint proximale (90) du joint (72) de manière à ouvrir un trajet de dérivation de joint (94) sur une étendue longitudinale de la lèvre de joint proximale (90-1) de la partie de joint proximale (90) du joint (72) de manière à établir à la fois un premier trajet d'air (94-1) au niveau de la première lumière intermédiaire (96) entre un diamètre extérieur de la canule sous vide (44) et un diamètre intérieur de la canule à stylet (46) et un second trajet d'air (94-2) au niveau de la seconde lumière intermédiaire (98) entre un diamètre intérieur de la canule de coupe (50) et le diamètre extérieur de la canule à stylet (46), le premier trajet d'air (94-1) et le second trajet d'air (94-2) étant en communication fluidique avec une région à proximité du joint (72).

2. Appareil de biopsie (10) selon la revendication 1, comprenant en outre un ensemble d'entraînement (12) ayant une source d'énergie électromécanique (20) et une source de vide (22), dans lequel l'ensemble sonde de biopsie (14) est fixé de manière amovible à l'ensemble d'entraînement (12).

3. Appareil de biopsie (10) selon la revendication 1, comprenant en outre un ensemble d'entraînement (12) ayant une source d'énergie électromécanique (20) et une source de vide (22), dans lequel la lumière sous vide (44-3) de la canule sous vide (44) est couplée en communication fluidique avec la source de vide (22).

4. Appareil de biopsie (10) selon la revendication 1, comprenant en outre un ensemble d'entraînement (12) ayant une source d'énergie électromécanique (20) et une source de vide (22), dans lequel la canule à stylet (46) est couplée en communication d'entraînement avec la source d'énergie électromécanique (20).

5. Appareil de biopsie (10) selon la revendication 1, comprenant en outre un ensemble d'entraînement (12) ayant une source d'énergie électromécanique (20) et une source de vide (22), dans lequel la canule de coupe (50) est couplée en communication d'entraînement avec la source d'énergie électromécanique (20).

6. Appareil de biopsie (10) selon la revendication 1, dans lequel la canule de coupe (50) est mobile par rapport à la canule à stylet (46) entre une seconde position étendue (70-2) pour couvrir une encoche d'échantillon (60) de la canule à stylet (46) et une seconde position rétractée (70-1) pour exposer l'encoche d'échantillon (60) lorsque la canule à stylet (46) est dans la première position étendue (68-2).

7. Appareil de biopsie (10) selon la revendication 1, dans lequel la canule à stylet (46) présente une partie proximale (46-1) et une partie distale (46-2), la partie distale (46-2) définissant une encoche d'échantillon (60).

8. Appareil de biopsie (10) selon la revendication 7, dans lequel la pluralité d'ouvertures d'évent (80) sont disposées à un emplacement à proximité de l'encoche d'échantillon (60).

9. Appareil de biopsie (10) selon la revendication 7, dans lequel le premier trajet d'air (94-1) et le second trajet d'air (94-2) convergent au niveau de l'encoche d'échantillon (60).

10. Appareil de biopsie (10) selon la revendication 1, dans lequel la pluralité d'ouvertures d'évent (80) incluent au moins deux fentes d'évent longitudinales (84).

11. Appareil de biopsie (10) selon la revendication 10, dans lequel une première fente d'évent longitudinale (82) et une seconde fente d'évent longitudinale (86) sont diamétralement opposées.

12. Appareil de biopsie (10) selon la revendication 1, dans lequel la pluralité d'ouvertures d'évent (80) incluent au moins un trou d'évent circulaire (88).

13. Appareil de biopsie (10) selon la revendication 12, dans lequel une extrémité proximale (82-1, 86-1) de l'au moins une fente d'évent longitudinale (82, 86) est alignée avec l'au moins un trou d'évent circulaire (88).
